# EUROPEAN PATENT APPLICATION

(11) **EP 3 878 432 A1**
(43) Date of publication of application: **15.09.2021**
(21) Application number: 19896530.3
(22) Date of filing: 13.12.2019
(51) Int. Cl.: A61K 9/00, A61K 35/28, A61P 1/16

(54) **PHARMACEUTICAL COMPOSITION FOR PREVENTING OR TREATING LIVER DISEASE, COMPRISING BIOIMPLANT COMPRISING MESENCHYMAL STEM CELLS**

(30) Priority: 14.12.2018 KR 20180162386
(71) Applicant: Thothscience Inc., Seoul 03995 (KR)
(72) Inventor: NOH, Seung Kwon, Seoul 03995 (KR); LEE, Chul Kyu, Seoul 03995 (KR)
(74) Representative: EP&C
(86) International application number: PCT/KR2019/017692
(87) International publication number: WO 2020/122666

(57) **Abstract**

The present invention relates to a pharmaceutical composition for preventing or treating liver diseases, comprising a bioimplant comprising mesenchymal stem cells, wherein the bioimplant comprising mesenchymal stem cells of the present invention can be inserted as a one-time subcutaneous or intraperitoneal implant in liver fibrosis animal models, to reduce the levels of AST and ALT, which are indicators of liver damage, as well as the ratio of an area occupied by collagen fibers in liver parenchyma, the number of degenerate hepatic cells, and the number of infiltrated inflammatory cells, and is effective in regenerating the liver, inhibiting liver fibrosis, and increasing the expression of growth factors, and thus can be beneficially used for preventing or treating liver diseases.

<110>     ThothScience Inc.

<120>     Pharmaceutical compostion for preventing or treating of liver
          disease comprising transplantation implant comprised mesenchymal
          stem cell

<130>     2019FPO-09-009/PCT

<150>     KR 1020180162386
<151>     2018-12-14

<160>     3

<170>     KoPatentIn 3.0

<210>     1
<211>     64
<212>     RNA
<213>     Artificial Sequence

<220>
<223>     miR-29(hsa-mir-20a)


<400>     1
augacugauu ucuuuuggug uucagaguca auauaauuuu cuagcaccau cugaaaucgg        60

uuau                                                                      64


<210>     2
<211>     110
<212>     RNA
<213>     Artificial Sequence

<220>
<223>     miR-34(hsa-mir-34a)


<400>     2
ggccagcugu gaguguuucu uuggcagugu cuuagcuggu uguugugagc aauaguaagg        60

aagcaaucag caaguauacu gcccuagaag ugcugcacgu uguggggccc                   110


<210>     3
<211>     22
<212>     RNA
<213>     Artificial Sequence

<220>
<223>     miR-150(hsa-miR-150-5p)


<400>     3
ucucccaacc cuuguaccag ug                                                  22

## Description

### BACKGROUND OF THE INVENTION

### 1. Field of the Invention

The present invention relates to a pharmaceutical composition for preventing or treating liver disease.

### 2. Description of the Related Art

Liver disease is caused by various reasons such as viral or bacterial infection, alcohol, various drugs, toxic chemicals, excessive accumulation of fat or heavy metals, and abnormal immune responses. Due to these reasons, viral hepatitis, alcoholic liver disease, non-alcoholic fatty liver, toxic hepatitis, and autoimmune liver disease occur. In addition, chronic liver disease that progresses slowly progresses to liver cirrhosis and liver cancer as liver fibrosis progresses gradually while not being aware of it. The cause of cirrhosis in Korea is mainly viral hepatitis or alcoholic liver disease. If liver damage is repeated continuously for any cause, there is a high risk of progressing to cirrhosis, liver fibrosis, or liver cancer regardless of the presence or absence of symptoms. Once cirrhosis occurs, it is difficult for the hardened liver to recover to its original state even after treatment.

Fatty liver is not a pathological condition by itself, and is a reversible symptom that naturally recovers when the causative substance is removed. However, if excessive fat accumulation in liver tissue is maintained continuously, steatohepatitis occurs, and as a result, hepatocyte necrosis and regeneration occur repeatedly, and in this process, fibrous extracellular matrix (ECM) increases, leading to liver fibrosis. In this process, endotoxemia, oxidative stress, and decreased liver regeneration ability play an important role. Particularly, it is known that neutrophil infiltration, necrosis, apoptosis, production of reactive oxygen species (ROS) and reactive nitrogen species (RNS), increase of proinflammatory cytokines and chemokines are involved (Bedossa P, Paradis V, J Pathol, 200, 504-515, 2003). In particular, hepatic stellate cells (HSC) are activated by various cytokines, growth factors and soluble mediators to synthesize collagen and accumulate in the extracellular matrix to increase fibrogenesis.

When liver damage reaches a certain stage and becomes chronic, the accumulation of ECM increases regardless of the type of causative agent, and regenerative nodules are formed as a result of continuous hepatocyte destruction and regeneration, leading to irreversible cirrhosis. However, despite many studies, the mechanism by which fatty liver, which is a relatively benign and reversible disease in the progression of liver disease, is deteriorated to irreversible liver fibrosis or cirrhosis is still not clearly identified. In addition, effective therapeutic agents for liver fibrosis and cirrhosis have not been developed until now.

Currently, treatment for liver fibrosis and cirrhosis is aimed at slowing the progression of symptoms and preventing the resulting decrease in liver function as much as possible. In the case of cirrhosis, depending on the cause, drugs such as Peginterferon or antiviral agents are used, and in severe cases, there is a method to reach a cure through liver transplantation. However, in the case of drug treatment, treatment resistance may appear, and in the case of liver transplantation, there are limitations in that there is a shortage of donors and the risk and cost associated with surgery, so the need for a new treatment method is emerging.

Liver regeneration therapy using stem cells has recently been receiving a lot of attention, and it is known that injected stem cells differentiate and reconstitute functional hepatocytes in the damaged liver, thereby exhibiting therapeutic effects. Until now, results of differentiation of stem cells into functional hepatocytes with high efficiency have been reported in vitro (Korean Patent No. 10-1542849). However, when stem cells are transplanted into animal models or humans, there is a problem that the efficiency of differentiation of hepatocytes is actually low. Moreover, since the exact markers of liver stem cells are not defined, there is a disadvantage that it is not possible to accurately know the stem cells that proliferate and differentiate into hepatocytes.

In addition, the biggest problem of the conventional stem cell treatment is that the in vivo engraftment rate and survival rate of cells are not constant depending on the patient, and thus accurate efficacy cannot be proved. And the possibility of stem cells becoming cancerous cannot be excluded. Therefore, there is a need to develop a new treatment method that can solve the problems of liver disease treatment using stem cells.

### SUMMARY OF THE INVENTION

It is an object of the present invention to provide a pharmaceutical composition for preventing or treating liver disease.

To achieve the above object, the present invention provides a pharmaceutical composition for preventing or treating liver diseases, comprising a bioimplant comprising mesenchymal stem cells.

### ADVANTAGEOUS EFFECT

The bioimplant comprising mesenchymal stem cells of the present invention can be inserted as a one-time subcutaneous or intraperitoneal implant in liver fibrosis animal models, to reduce the levels of AST and ALT, which are indicators of liver damage, as well as the ratio of an area occupied by collagen fibers in liver parenchyma, the number of degenerate hepatic cells, and the number of infiltrated inflammatory cells, and is effective in regenerating the liver, inhibiting liver fibrosis, and increasing the expression of growth factors, and thus can be beneficially used for preventing or treating liver disease.

### BRIEF DESCRIPTION OF THE DRAWINGS

Figure 1 is a graph illustrating the results of measuring the concentration of HGF secreted according to the incubation time of a bioimplant (hereinafter referred to as INBS-ASC-Ther) comprising adipose-derived stem cells (ASCs).
Figure 2 is a graph illustrating the results of measuring the concentration of VEGF secreted according to the incubation time of INBS-ASC-Ther.
Figure 3 is a graph illustrating the serum AST levels measured on days 1, 5, 12 and 18 after the administration of INBS-ASC-Ther to a liver fibrosis animal model.
   (G1: normal group;
   G2 ∼ G4: experimental groups with liver damage induced by DMN administration;
   G2: non-treated group;
   G3: INBS-ASC-Ther-treated group cultured in hypoxic conditions; and
   G4: INBS-ASC-Ther-treated group not cultured in hypoxic conditions).
Figure 4 is a graph illustrating the serum ALT levels measured on days 1, 5, 12 and 18 after the administration of INBS-ASC-Ther to a liver fibrosis animal model.
Figure 5 is a graph illustrating the serum TG levels measured on days 1, 5, 12 and 18 after the administration of INBS-ASC-Ther to a liver fibrosis animal model.
Figure 6 is a set of photographs illustrating the images of the liver tissues extracted on the 18^{th} day after the administration of INBS-ASC-Ther to a liver fibrosis animal model, stained with H&E (hematoxylineosin) and Masson's trichrome.
   (H&E: X40; Masson's trichrome: X200;
   A: G1 (normal group);
   B: G2 (non-treated group);
   C: G3 (INBS-ASC-Ther-treated group cultured in hypoxic conditions);
   D: G4 (INBS-ASC-Ther-treated group not cultured in hypoxic conditions);
   CV: central vein;
   PT: portal triad region; and
   Scale bar: 100 *µ*m).

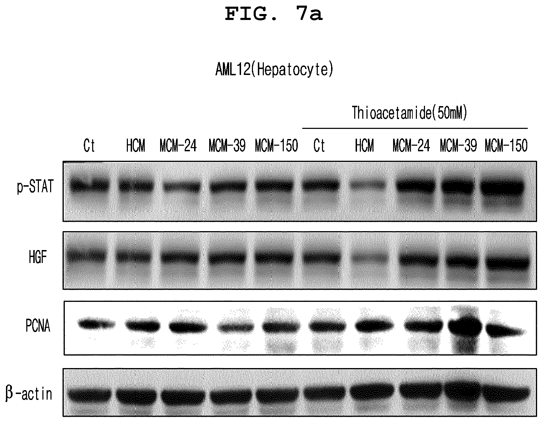

Figure 7a is a diagram illustrating the expressions of p-STAT, HGF and PCNA, which are factors involved in liver regeneration, involved in cell proliferation in a liver cell line when the micro RNA-introduced ASC-enriched culture medium is treated, measured by Western blotting.

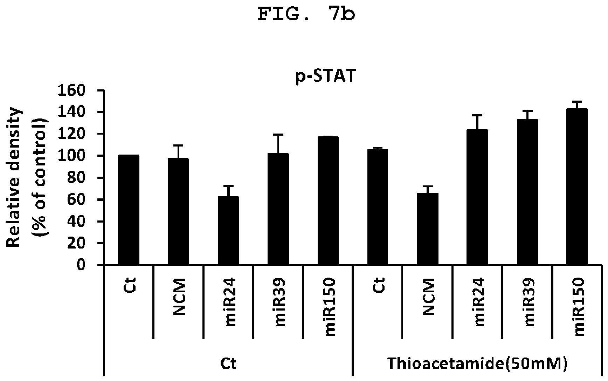

Figure 7b is a graph confirming that the expression of p-STAT, a factor related to liver regeneration, is increased in a liver cell line when the micro RNA-introduced ASC-enriched culture medium is treated.

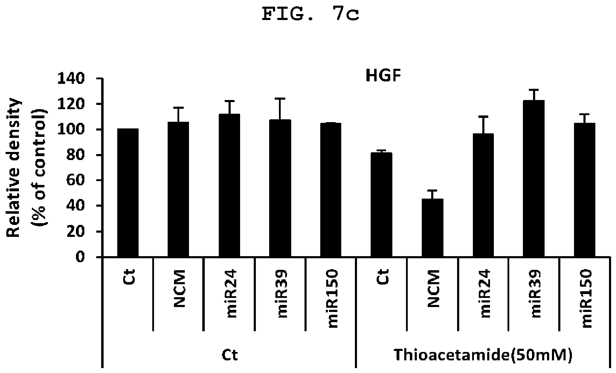

Figure 7c is a graph confirming that the expression of HGF, a factor related to liver regeneration, is increased in a liver cell line when the micro RNA-introduced ASC-enriched culture medium is treated.

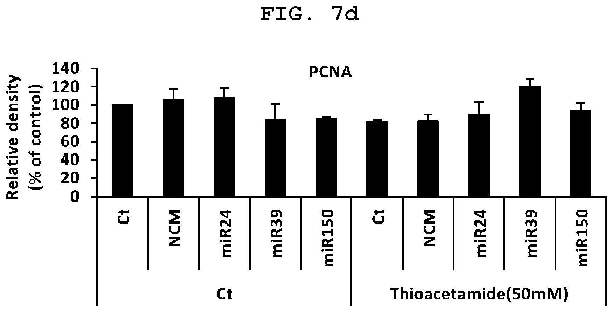

Figure 7d is a graph confirming that the expression of PCNA, a factor related to liver regeneration, is increased in a liver cell line when the micro RNA-introduced ASC-enriched culture medium is treated.

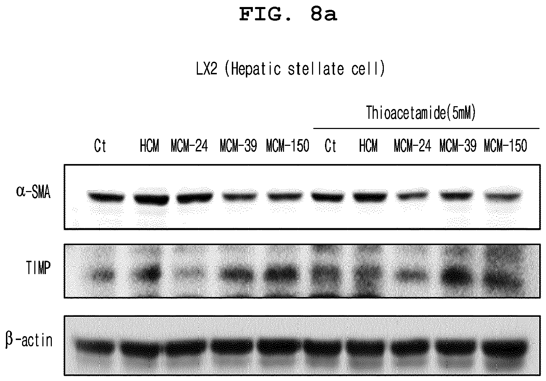

Figure 8a is a diagram illustrating the expressions of α-SMA, a factor related to liver fibrosis, and TIMP, a fibrosis inhibitory factor, in a hepatic stellate cell line when the micro RNA-introduced ASC-enriched culture medium is treated, measured by Western blotting.

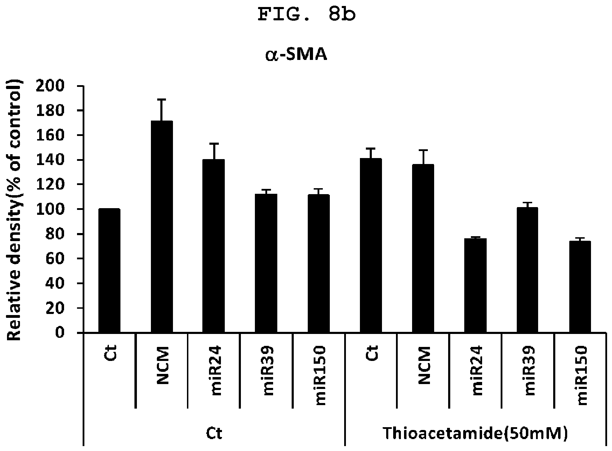

Figure 8b is a graph confirming that the expression of α-SMA, a factor related to liver fibrosis, is reduced in a hepatic stellate cell line when the micro RNA-introduced ASC-enriched culture medium is treated.

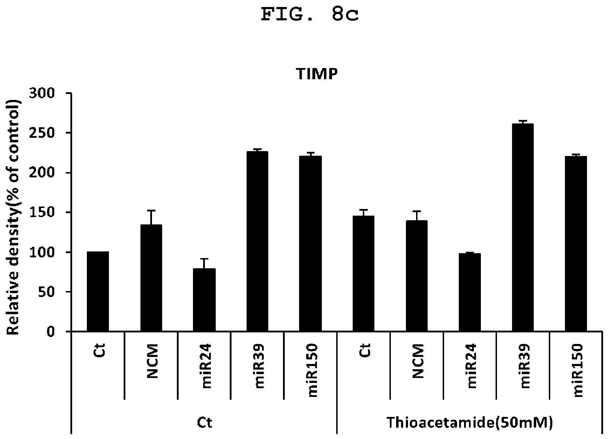

Figure 8c is a graph confirming that the expression of TIMP, a fibrosis inhibitory factor, is increased in a hepatic stellate cell line when the micro RNA-introduced ASC-enriched culture medium is treated.

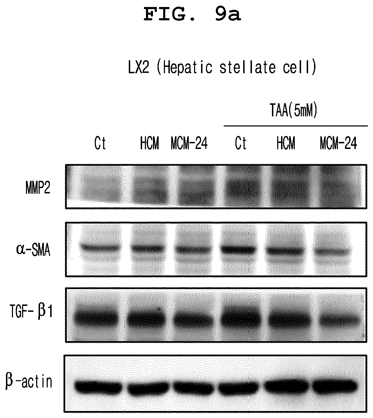

Figure 9a is a diagram illustrating the expressions of α-SMA, MMP2 and TGF-β1, which are factors involved in liver fibrosis, in a hepatic stellate cell line when the miR24-introduced ASC-enriched culture medium is treated, measured by Western blotting.

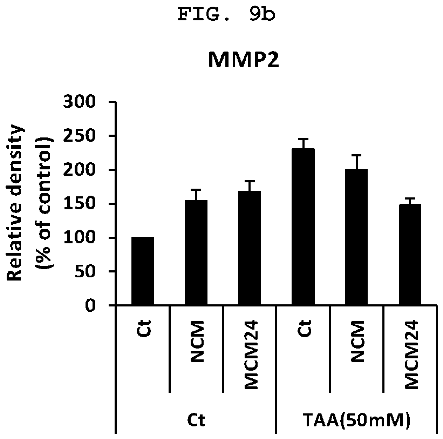

Figure 9b is a graph confirming that the expression of MMP2 is reduced in a hepatic stellate cell line when the miR24-introduced ASC-enriched culture medium is treated.

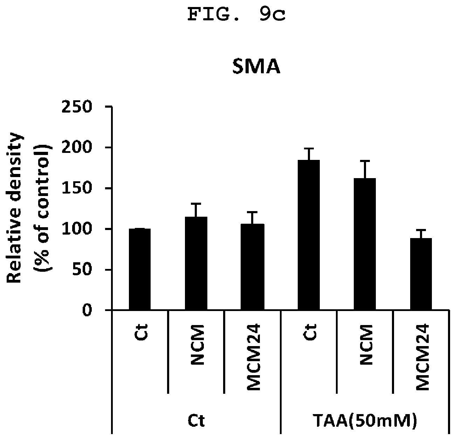

Figure 9c is a graph confirming that the expression of SMA is reduced in a hepatic stellate cell line when the miR24-introduced ASC-enriched culture medium is treated.

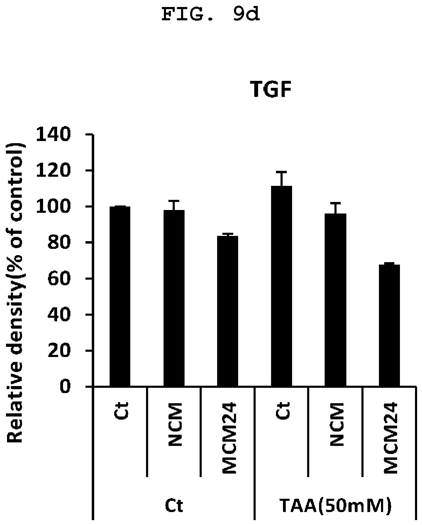

Figure 9d is a graph confirming that the expression of TGF is reduced in a hepatic stellate cell line when the miR24-introduced ASC-enriched culture medium is treated.

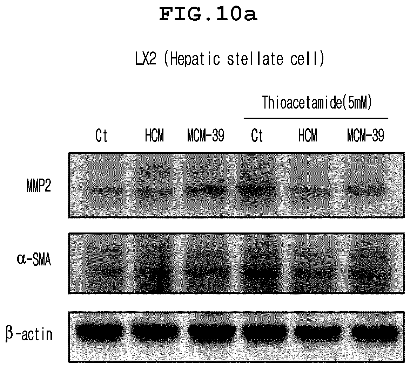

Figure 10a is a diagram illustrating the expressions of MMP2 and α-SMA, which are factors involved in liver fibrosis, in a hepatic stellate cell line when the miR39-introduced ASC-enriched culture medium is treated, measured by Western blotting.

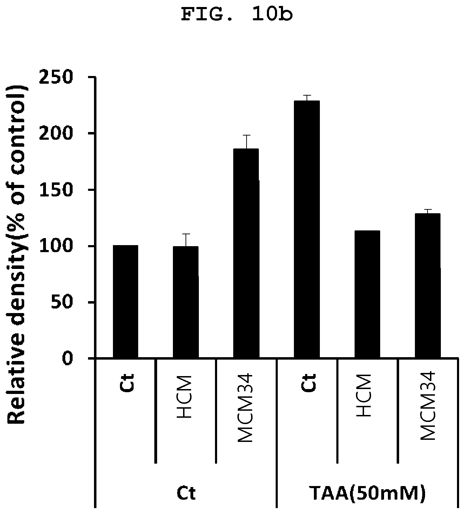

Figure 10b is a graph confirming that the expression of MMP2 is reduced in a hepatic stellate cell line when the miR39-introduced ASC-enriched culture medium is treated.

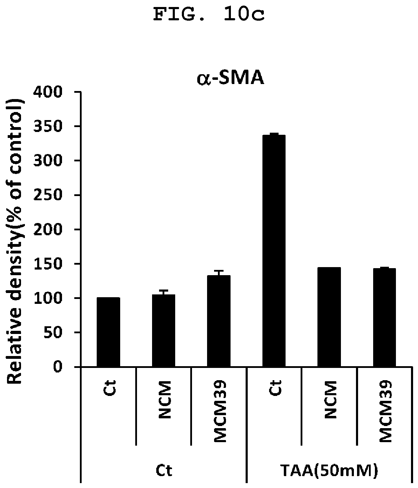

Figure 10c is a graph confirming that the expression of α-SMA is reduced in a hepatic stellate cell line when the miR39-introduced ASC-enriched culture medium is treated.

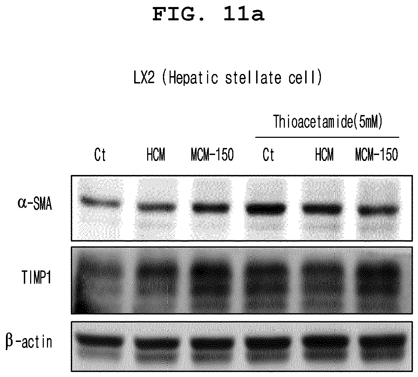

Figure 11a is a diagram illustrating the expressions of α-SMA and TIMP-1, which are factors involved in liver fibrosis, in a hepatic stellate cell line when the miR150-introduced ASC-enriched culture medium is treated, measured by Western blotting.

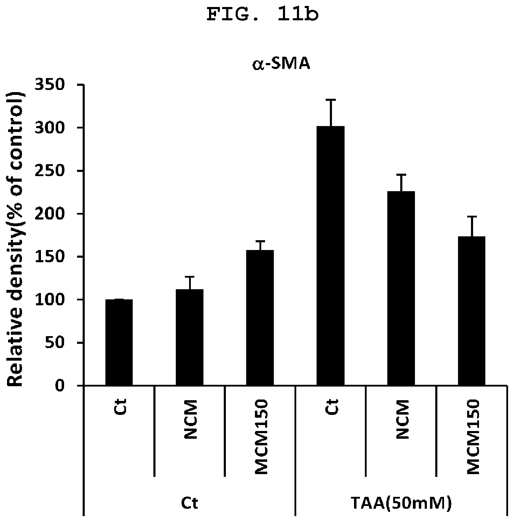

Figure 11b is a graph confirming that the expression of α-SMA is reduced in a hepatic stellate cell line when the miR150-introduced ASC-enriched culture medium is treated.

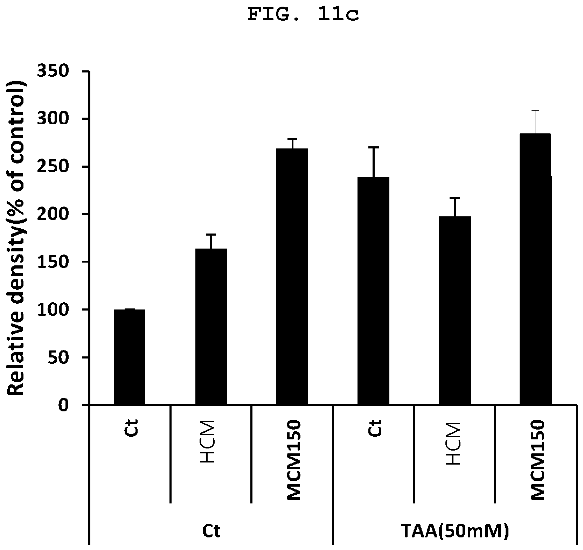

Figure 11c is a graph confirming that the expression of TIMP-1 is increased in a hepatic stellate cell line when the miR150-introduced ASC-enriched culture medium is treated.

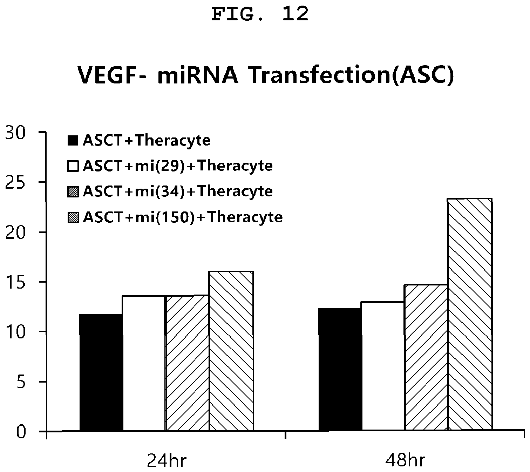

Figure 12 is a graph confirming that the secretion of VEGF, which is a growth factor, is increased in a bioimplant comprising ASCs introduced with miR24, a bioimplant comprising ASCs introduced with miR39, and a bioimplant comprising ASCs introduced with miR150, compared to a bioimplant comprising ASC.

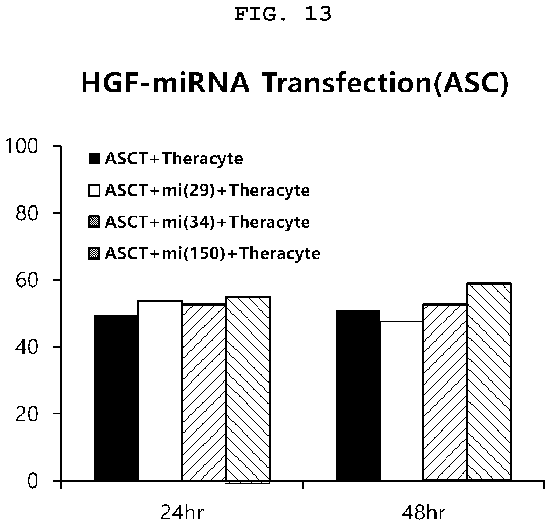

Figure 13 is a graph confirming that the secretion of HGF, which is a growth factor, is increased in a bioimplant comprising ASCs introduced with miR24, a bioimplant comprising ASCs introduced with miR39, and a bioimplant comprising ASCs introduced with miR150, compared to a bioimplant comprising ASC.

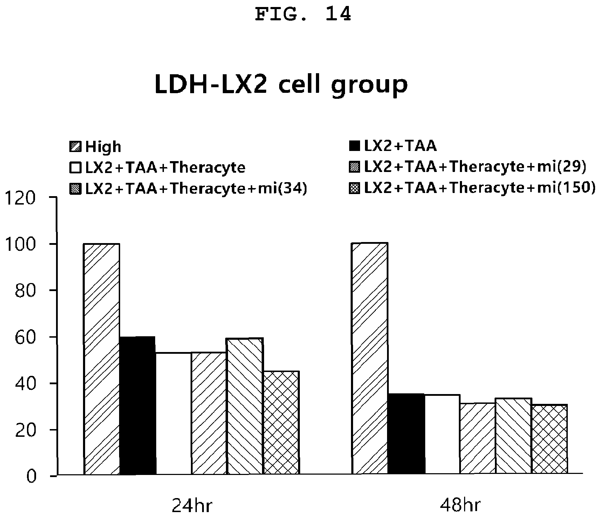

Figure 14 is a graph confirming that the secretion of lactate dehydrogenase (LDH), which is a liver disease marker, is reduced in a bioimplant comprising ASCs introduced with miR24, a bioimplant comprising ASCs introduced with miR39, and a bioimplant comprising ASCs introduced with miR150, compared to a bioimplant comprising ASCs.

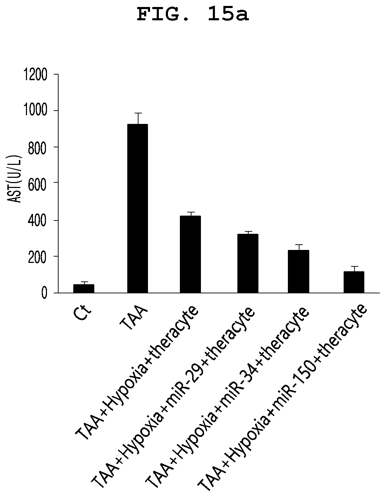

Figure 15a is a graph illustrating that the AST level is decreased in the group in which a bioimplant comprising ASCs introduced with miR24, miR39 or miR150 is intraperitoneally administered compared to the group in which liver fibrosis is induced by treating TAA alone.

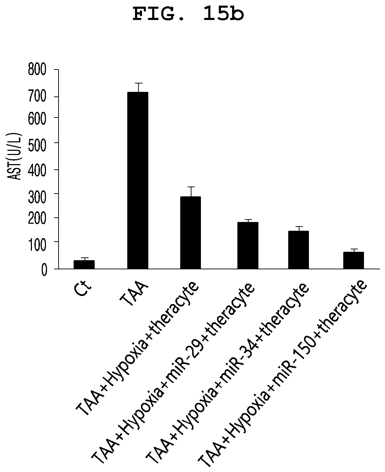

Figure 15b is a graph illustrating that the ALT level is decreased in the group in which a bioimplant comprising ASCs introduced with miR24, miR39 or miR150 is intraperitoneally administered compared to the group in which liver fibrosis is induced by treating TAA alone.

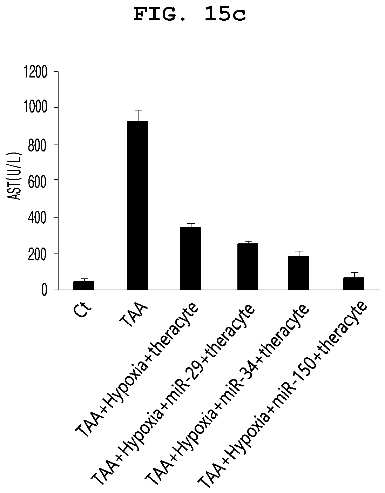

Figure 15c is a graph illustrating that the AST level is decreased in the group in which a bioimplant comprising ASCs introduced with miR24, miR39 or miR150 is subcutaneously administered compared to the group in which liver fibrosis is induced by treating TAA alone.

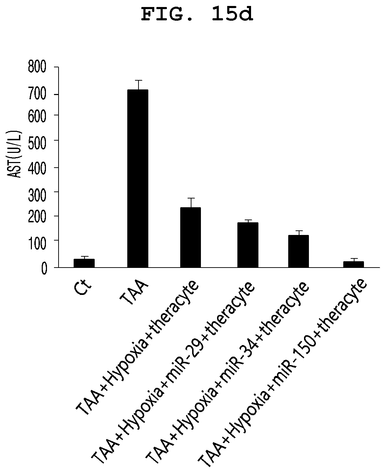

Figure 15d is a graph illustrating that the ALT level is decreased in the group in which a bioimplant comprising ASCs introduced with miR24, miR39 or miR150 is subcutaneously administered compared to the group in which liver fibrosis is induced by treating TAA alone.

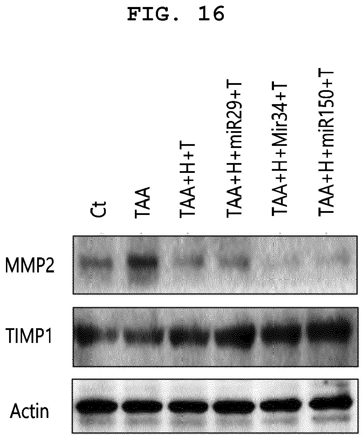

Figure 16 is a diagram illustrating the expressions of MMP2, a liver fibrosis-inducing factor, and TIMP1, a liver fibrosis inhibitory factor, in the group in which a bioimplant comprising ASCs introduced with miR24, miR39 or miR150 is administered compared to the group in which liver fibrosis is induced by treating TAA alone, measured by Western blotting. The group in which a bioimplant comprising ASCs introduced with miR24, miR39 or miR150 is administered shows an antifibrotic effect by adjusting the fibrosis factors by TAA by decreasing the expression of MMP2 and increasing the expression of TIMP1.

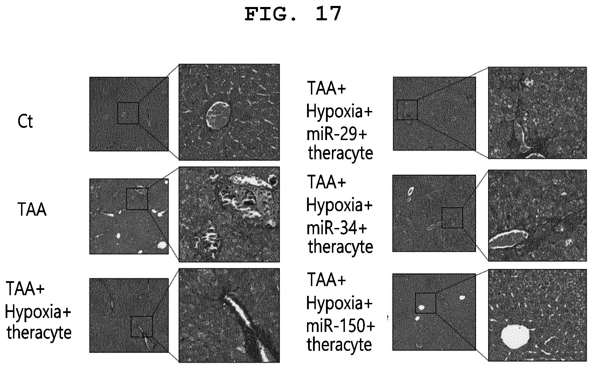

Figure 17 is a set of photographs confirming the degree of fibrosis of liver tissue in the experimental group through H&E and Mason's trichrome staining. Compared to the group in which liver fibrosis is induced by treating TAA alone, the expression of MMP2 is decreased in the group in which a bioimplant comprising ASCs introduced with miR24, miR39 or miR150 is intraperitoneally administered, resulting in a decrease in TAA-induced fibrin.

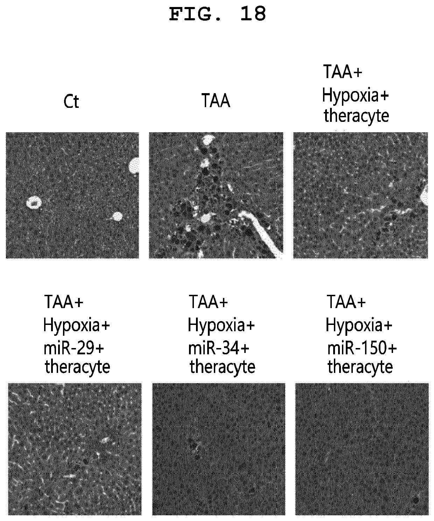

Figure 18 is a set of photographs confirming the expression of MMP2 in the experimental group through immunohistochemical staining. Compared to the group in which liver fibrosis is induced by treating TAA alone, the expression of MMP2, a liver fibrosis-inducing factor, is decreased in the group in which a bioimplant comprising ASCs introduced with miR24, miR39 or miR150 is intraperitoneally administered, resulting in a decrease in TAA-induced fibrin.

### DESCRIPTION OF THE PREFERRED EMBODIMENTS

Hereinafter, the present invention is described in detail.

The present invention provides a pharmaceutical composition for preventing or treating liver diseases, comprising a bioimplant comprising mesenchymal stem cells.

The bioimplant can be a thin film polymer chamber made of an in vivo biocompatible membrane. The bioimplant is a container for in vivo implantation, that is, a transplantation container, and has a space for injecting stem cells, and substances produced by stem cells, particularly cytokines, can be secreted out of the implant through a thin film. The bioimplant protects stem cells from rejection by the recipient, and can have an angiogenesis function that induces the formation of capillaries close to a membrane when implanted subcutaneously. Through this angiogenesis function, the bioimplant can provide nutrients and blood to stem cells in the thin film. The bioimplant can be made of a bio-friendly material, that is, a material that does not induce an immune response, such as polyurethane, but not always limited thereto. The bioimplant can have a porous surface in which a plurality of micropores is located on the surface. At this time, the pores can have a size that does not allow large substances such as cells to pass through, but a size that allows proteins or nutrients to pass through. Therefore, the stem cells injected into the implant through the pores cannot come out of the implant, and thus cancer induction due to the stem cells is prevented. On the other hand, growth promoting factors, which are secretions of stem cells, are released into the blood to form blood vessels around them, so that oxygen and nutrients can be supplied into the implant. The bioimplant can be easily removed even after a long time after transplantation. The bioimplant can be used by purchasing a commercially available product that satisfies the above conditions, or manufactured and used, and specifically may be TheraCyte™. As the bioimplant, a commercially available product that satisfies the above conditions can be purchased or manufactured, and the bioimplant can be specifically TheraCyte™.

The mesenchymal stem cells can be adipose-derived stem cells (ASCs), but not always limited thereto.

The mesenchymal stem cells can secrete factors capable of preventing or treating liver disease. The mesenchymal stem cells can be protected from rejection reactions to stem cells by being included in the bioimplant. The mesenchymal stem cells are self-renewing without being scattered after administration to a living body, accumulating in the implant, and responding to the signal of the liver damage of the living body, and can more continuously secrete factors capable of preventing or treating liver disease. In addition, it is possible to obtain stem cells more suitable for the treatment or prevention of liver diseases by adjusting the culture conditions of the mesenchymal stem cells.

In the bioimplant, 1×10² to 1×10¹² cells of the mesenchymal stem cells can be included. Particularly, 1×10⁴ to 1×10¹⁰ cells, and more particularly 1×10⁵ to 1×10⁹ cells can be included, but not always limited thereto.

The liver disease can be any one or more selected from the group consisting of hepatitis, liver fibrosis and cirrhosis.

The mesenchymal stem cells are the mesenchymal stem cells into which miRNA (micro RNA) has been introduced.

The said miRNA refers to non-coding RNA that regulates the expressions of various genes, and can inhibit the gene expression in the post-transcriptional stage or induce the target RNA degradation by suppressing the translation of mRNA or inducing the degradation of mRNA. Post-transcriptional regulation is used as a powerful regulation method in processes that require the accurate and precise gene expression such as intracellular signaling. Since about half of the changes in gene expression caused by external stimuli are regulated in the post-transcriptional stage, it is reported that post-transcriptional regulation plays a very important role in the regulation of the expression of the entire gene. Several studies have shown that miRNA plays an important role in controlling many biological processes such as cell differentiation, proliferation, apoptosis, development, immunity, metabolism and stem cell maintenance. Unlike peptides or antibody therapeutics, miRNA has the advantage of having a short development period, and theoretically all therapeutics can be developed if only certain disease target gene is identified. In addition, miRNA generally has very high specificity and efficacy by simultaneously regulating the expressions of multiple target genes rather than one.

The miRNA can be miR24, miR39 or miR150, but not always limited thereto.

The composition increases the expression of PCNA, HGF or p-STAT3.

The PCNA, HGF or p-STAT3 is a factor related to liver regeneration, and when its expression is increased, it can be determined that the damaged liver cells are improved.

The composition decreases the expression of α-SMA, MMP, TGF-β1 or TIMP-1.

The α-SMA, MMP, TGF-β1 or TIMP-1 is a liver fibrosis inducer, and when its expression is reduced, it can be determined that liver fibrosis is suppressed.

The composition increases the amount of secretion of vascular endothelial growth factor (VEGF) or hepatocyte growth factor (HGF).

When the amount of secretion of vascular endothelial growth factor (VEGF) or hepatocyte growth factor (HGF) is increased, it can be determined that the damaged liver tissue has been regenerated.

The composition reduces the amount of secretion of lactate dehydrogenase (LDH).

When cells are destroyed, the level of lactate dehydrogenase (LDH) is increased in the blood, and the activity of lactate dehydrogenase (LDH) is often high in malignant tumors, liver diseases, heart diseases, and blood diseases. In the case that the amount of LDH secretion was increased due to liver disease, it can be determined that liver disease has improved when the amount of LDH secretion is reduced.

The composition can reduce the ALT level increased by liver damage. In addition, the composition can reduce the ratio of an area occupied by collagen fibers in liver parenchyma, and can reduce the number of degenerate hepatic cells or the number of infiltrated inflammatory cells.

The composition can be injected subcutaneously. Particularly, it can be injected subcutaneously into the back, but the injection site is not limited.

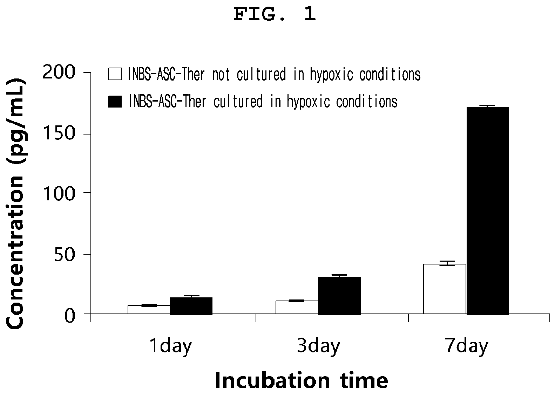

In a preferred embodiment of the present invention, adipose-derived stem cells were injected into TheraCyte™ (hereinafter referred to as INBS-ASC-Ther), which was inserted subcutaneously into the back of a liver fibrosis animal model to perform blood biochemical tests, autopsies, and histopathological tests. As a result, the INBS-ASC-Ther experimental group cultured under hypoxic conditions showed no difference in blood biochemical and histopathological tests compared to the untreated group. In blood biochemical tests, a tendency to decrease ALT levels, an indicator of liver damage, was confirmed in the INBS-ASC-Ther experimental group not cultured under hypoxic conditions. In histopathological tests, it was confirmed that the ratio of an area occupied by collagen fibers in liver parenchyma, the number of degenerate hepatic cells, and the number of infiltrated inflammatory cells were significantly decreased (see Figures 1 to 6).

In addition, INBS-ASC-Ther can have an effect on improving liver function even if it is injected subcutaneously into the back, which is a site far away from the liver, a disease region.

The present inventors confirmed that the expressions of p-STAT3, HGF and PCNA, which are factors involved in liver regeneration, were increased (see Figures 7a to 7d), the expression of α-SMA, which is a factor involved in liver fibrosis, was reduced, and the expression of TIMP, a fibrosis inhibitory factor, was increased (see Figures 8a to 8c) when a liver cell line was treated with the three types of miRNA-introduced adipose-derived stem cell-enriched culture medium. In addition, it was confirmed that the expressions of α-SMA and MMP2, which are factors involved in liver fibrosis, were reduced when miR24-introduced adipose-derived stem cells, miR39-introduced adipose-derived stem cells, and miR150-introduced adipose-derived stem cells were treated to hepatic stellate cells, respectively (see Figures 9a to 11c). It was also confirmed that the micro RNA-introduced adipose-derived stem cells in the bioimplant produced by injecting miR24, miR39 or miR150-introduced adipose-derived stem cells into TheraCyte™ (miR24-ASC-Theracyte, miR39-ASC-Theracyte, miR150-ASC-Theracyte) increased the secretion of vascular epithelial growth factor (VEGF) and hepatocyte growth factor (HGF), and reduced the secretion of lactate dehydrogenase (LDH) (Figures 12 to 14).

In addition, when a bioimplant comprising miR24, miR39 or miR150-introduced ASCs was administered intraperitoneally or subcutaneously, the effect of reducing the levels of AST and ALT was confirmed (Figures 15a to 15d). It was also confirmed that the group in which a bioimplant comprising ASCs introduced with miR24, miR39 or miR150 was administered showed an antifibrotic effect by adjusting the fibrosis factors by TAA by decreasing the expression of MMP2 and increasing the expression of TIMP1 compared to the group in which liver fibrosis was induced by treating TAA alone (Figure 16).

In addition, it was confirmed that the expression of MMP2 was decreased in the group in which a bioimplant comprising ASCs introduced with miR24, miR39 or miR150 was intraperitoneally administered, resulting in a decrease in TAA-induced fibrin, compared to the group in which liver fibrosis was induced by treating TAA alone (Figures 17 and 18).

Therefore, the bioimplant comprising mesenchymal stem cells or the bioimplant comprising mi-RNA-introduced mesenchymal stem cells of the present invention can be effectively used for preventing or treating liver diseases.

The pharmaceutical composition according to the present invention can contain the bioimplant comprising mesenchymal stem cells, the active ingredient, at the concentration of 10 ∼ 95 weight% by the total weight of the composition. In addition, the pharmaceutical composition of the present invention can further include one or more active ingredients having the same or similar function in addition to the active ingredient described above.

The pharmaceutical composition of the present invention can include carriers, diluents, excipients or mixtures thereof commonly used in biological preparations. The pharmaceutically acceptable carrier can be any carrier that is able to deliver the composition of the present invention in the living body without limitation, which is exemplified by the compounds described in Merck Index, 13th ed., Merck & Co. Inc., such as saline, sterilized water, Ringer's solution, dextrose solution, maltodextrin solution, glycerol, ethanol, or a mixture thereof. If necessary, a general additive such as antioxidant, buffer, and bacteriostatic agent can be additionally added.

When formulating the composition, generally used diluents or excipients such as fillers, extenders, binders, wetting agents, disintegrating agents and surfactants can be added.

The composition of the present invention can be formulated as an oral or parenteral preparation. Oral preparations can include solid formulations and liquid formulations. The solid formulation can be tablets, pills, powders, granules, capsules or troches. Such solid formulation can be prepared by adding at least one excipient to the composition. The excipient can be starch, calcium carbonate, sucrose, lactose, gelatin, or a mixture thereof. In addition, the solid preparation can contain lubricants such as magnesium stearate and talc. The liquid formulation can be suspensions, solutions, emulsions or syrups. In this case, the liquid formulation can contain excipients such as wetting agents, sweetening agents, fragrances, and preservatives.

The parenteral preparation can include injections, suppositories, powders for respiratory inhalation, spray aerosols, powders and creams. The injection can include sterilized aqueous solutions, non-aqueous solvents, suspensions, emulsions, and the like. At this time, as the non-aqueous solvent or suspension, vegetable oils such as propylene glycol, polyethylene glycol and olive oil, or injectable esters such as ethyl oleate can be used.

The composition of the present invention can be administered orally or parenterally according to a desired method. Parenteral administration can include intraperitoneal injection, rectal injection, subcutaneous injection, intravenous injection, intramuscular injection or intrathoracic injection.

The composition can be administered by the pharmaceutically effective amount. The effective amount can be determined according to the type of disease, the severity, the activity of the drug, the patient's sensitivity to the drug, the time of administration, the route of administration, the duration of treatment, the drugs being used simultaneously, and the like. However, for the desired effect, the amount of mesenchymal stem cells included in the pharmaceutical composition according to the present invention can be 1×10² ∼ 1×10¹² cells/time, preferably 1×10⁴ ∼ 1×10¹⁰ cells/time, and more preferably 1×10⁵ ∼ 1×10⁹ cells/time, but is not limited thereto. The administration frequency is once a day or a few times a day.

The composition of the present invention can be administered alone or in combination with other therapeutic agents. In combination administration, the administration can be sequential or simultaneous.

### Detailed description of several examples

Hereinafter, the present invention will be described in detail by the following examples.

However, the following examples are only for illustrating the present invention, and the contents of the present invention are not limited thereto.

### Example 1: Culture of adipose-derived stem cells (ASCs)

Adipose-derived stem cells (ASCs) were separated from the adipose tissue obtained from a healthy person in an operation room by performing washing, micing, digestion, neutralization, centrifugation, and filtration according to the general method of separating adipose-derived stem cells, and cultured. The morphology of the cells was confirmed by flow cytometry. Then, subculture was performed to secure a large amount of adipose-derived stem cells (adipose stem cells). The obtained adipose stem cells were cultured in a T75 flask containing DMEM (Low glucose, 10% FBS, 1% P/S) until they were 70 to 80% full and used in the following experiment.

### Example 2: Preparation of adipose-derived stem cells contained in bioimplant (INBS-ASC-Ther)

The adipose-derived stem cells cultured in Example 1 were injected into the bioimplant TheraCyte™.

Particularly, the adipose-derived stem cells cultured in Example 1 were divided into two experimental groups. In one experimental group, 1×10⁷ adipose-derived stem cells cultured in Example 1 were injected into TheraCyte™ (TheraCyte Inc., PD20.0, Ported TheraCyte® Device). In the other experimental groups, 1×10⁷ adipose-derived stem cells cultured for 24 hours in a hypoxia chamber were injected into TheraCyte™ in the same manner.

### Experimental Example 1: Measurement of HGF and VEGF secretion in adipose-derived stem cells contained in bioimplant (INBS-ASC-Ther)

The INBS-ASC-Thers of the two experimental groups prepared in Example 2 were placed so as to be immersed in a 100 mm plate containing DMEM (Low glucose, 10% FBS, 1% P/S) and cultured for 7 days. 500 µl of the culture solution was taken on the 1^{st}, 3^{rd} and 7^{th} days of culture, and the secretion amounts of HGF and VEGF were measured using an ELISA kit, respectively.

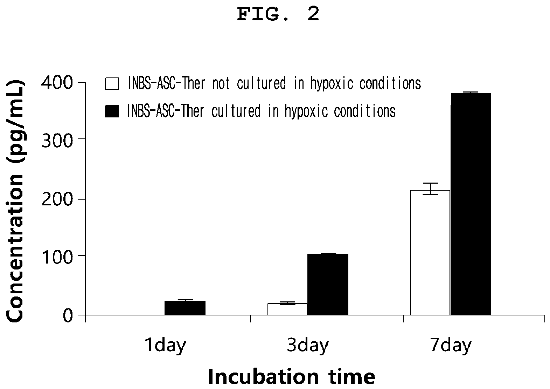

As a result, both INBS-ASC-Thers of the two experimental groups secreted HGF and VEGF, and the most HGF or VEGF was secreted on the 7^{th} day. In addition, INBS-ASC-Ther increased the secretion of HGF and VEGF when cultured in hypoxia (Figures 1 and 2).

### Experimental Example 2: Construction of liver fibrosis animal model

SD rats (male) purchased from OrientBio were acclimated for 5 days, and DMN (dimethylnitrosamine) was administered intraperitoneally to the animals at a dose of 10 mg/kg 3 times a week for 3 consecutive days for a total of 3 weeks. Blood was collected 2 weeks after the DMN administration, and the level of liver ALT (alanine aminotransferase) was confirmed with the collected blood serum. The following experiment was conducted by selecting the animals with liver fibrosis induced above a certain level.

### Experimental Example 3: Therapeutic effect of adipose-derived stem cells contained in bioimplant on liver fibrosis animal model

In Experimental Example 2, the experimental groups were randomly divided so that the mean value of each group was uniform according to the result of the ALT value. The experimental groups were the normal group without DMN administration (G1) and the experimental group administered with DMN. The experimental group administered with DMN was further divided into the non-treated group (G2), the group administered with INBS-ASC-Ther cultured in hypoxia (G3), and the group administered with INBS-ASC-Ther (G4). In the G3 and G4 experimental groups, 1 INBS-ASC-Ther comprising 1×10⁷ hypoxic-treated or non-treated adipose-derived stem cells was injected subcutaneously on the back of the mouse 5 days after the termination of the second week of DMN administration. Then, clinical pathology tests, autopsy and histopathological tests for each experimental group were performed.

### <3-1> Blood biochemical tests

Blood was collected on the 1^{st}, 5^{th}, 12^{th}, and 18^{th} days of the administration of hypoxic-treated or non-treated INBS-ASC-Ther, and blood biochemical tests were performed for each experimental group. In the case of the 1^{st} day, the tests were performed 1 hour after the administration of hypoxic-treated or non-treated INBS-ASC-Ther. Particularly, about 700 µl of blood was obtained each time from the jugular vein of each animal using a syringe, and the obtained blood was injected into a vacutainer tube containing a clot activator, and then coagulated at room temperature for 30 minutes. The coagulated blood was centrifuged at 3,000 rpm for 10 minutes to separate the serum, and the serum was stored frozen until analysis. On the day of autopsy (the 18^{th} day), the animal model was anesthetized by inhaling isoflurane, and blood was collected from the postcaval vein using a syringe. The levels of AST (aspartate aminotransferase), ALT (alanine aminotransferase) and TG (triglyceride) in the obtained serum were measured.

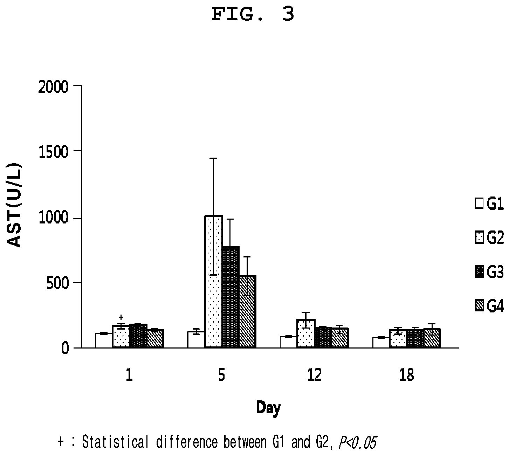

As a result of the AST measurement, it was confirmed that the level of AST was significantly increased in the non-treated group (G2), compared to the normal group (G1) on day 1 (P<0.05). There was no statistical significance on other measurement days, but an increasing trend was observed. In the hypoxic-treated or non-treated INBS-ASC-Ther groups (G3 and G4), there was no statistical difference compared to the non-treated group (G2), but a decreasing trend was observed until day 12 (Figure 3).

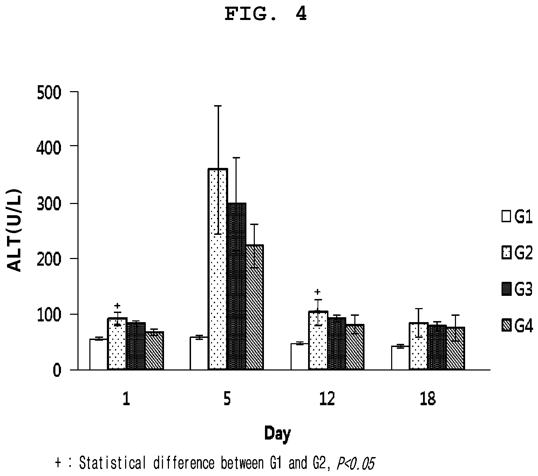

As a result of the ALT measurement, it was confirmed that the level of ALT was significantly increased in the non-treated group (G2), compared to the normal group (G1) on day 1 and day 12 (P<0.05). There was no statistical significance on other measurement days, but an increasing trend was observed. In the hypoxic-treated group (G3), there was no statistical difference compared to the non-treated group (G2). In the hypoxic-non-treated INBS-ASC-Ther groups (G4), there was no statistical difference compared to the non-treated group (G2), but a decreasing trend was observed (Figure 4).

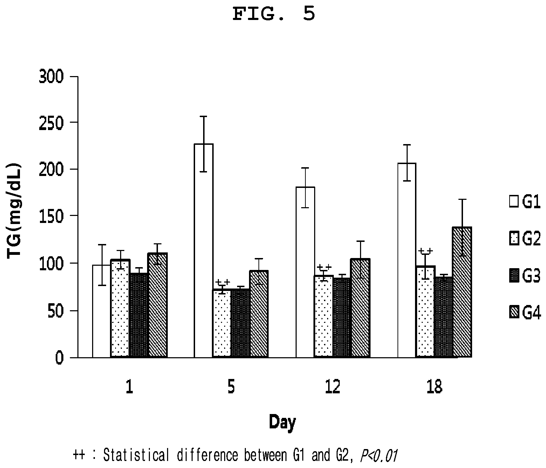

As a result of the TG measurement, it was confirmed that the level of TG was significantly reduced in the non-treated group (G2), compared to the normal group (G1) on day 5, day 12 and day 18 (P<0.01). In the hypoxic-treated or non-treated INBS-ASC-Ther groups (G3 and G4), there was no statistical difference compared to the non-treated group (G2), but it was confirmed that the level was increased to the level of the normal group (G1) group except for day 1 (Figure 5).

From the above results, it was confirmed that the AST and ALT levels, which were highly related to liver function, were significantly increased in the non-treated group (G2) compared to the normal group (G1), indicating that liver damage was induced by DMN. In the hypoxic-treated INBS-ASC-Ther group (G3), compared to the non-treated group (G2), the AST and ALT levels, the indices of liver damage, tended to decrease, but the levels did not show any difference on the day of autopsy, indicating that there was no improvement in liver damage. In the hypoxic-non-treated INBS-ASC-Ther group (G4), the level of AST showed a tendency to decrease compared to the non-treated group (G2), but the level did not show any difference on the day of autopsy, but the level of ALT showed a tendency to decrease continuously, indicating that liver damage was improved.

### <3-2> Histopathological tests

For histopathological tests for each experimental group, blood was collected on the 18^{th} day of administration of hypoxic-treated or non-treated INBS-ASC-Ther, and then the abdominal artery and postcaval vein were cut to bleed/kill. The liver was extracted from the animal and fixed in 10% neutral buffered formalin fixative. The fixed tissues were commissioned to College of Korean Medicine for histopathological tests using H&E and Masson's trichrome staining. Particularly, collagen fiber occupied regions, degenerative hepatocyte numbers and inflammatory cell numbers were measured. Eclipse 80i (Nikon, Japan) was used as a microscope, and iSolution FL ver 9.1 (iSolution, Inc., Canada) program was used for analysis.

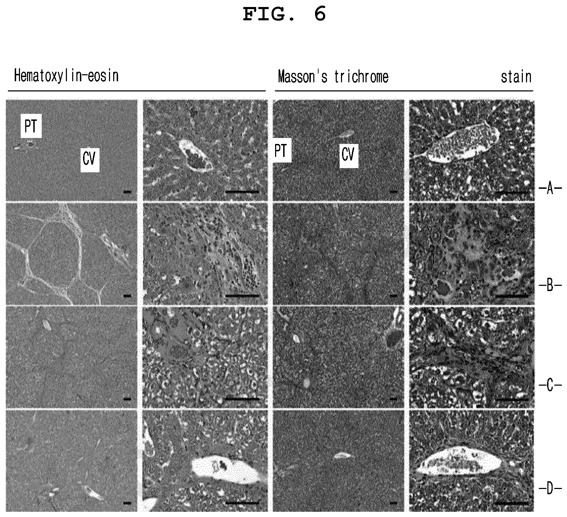

As a result, in the non-treated group (G2), the ratio of an area occupied by collagen fibers in liver parenchyma, the number of degenerate hepatic cells, and the number of infiltrated inflammatory cells were significantly increased (P<0.01) compared to the normal group (G1). Therefore, it was confirmed that liver damage was induced by DMN. In the hypoxic-treated INBS-ASC-Ther group (G3), there was no difference in all the measurement items compared to the non-treated group (G2), whereas in the hypoxic-non-treated INBS-ASC-Ther group (G4), all the measurement items were significantly decreased (P<0.05 or P<0.01) compared to the non-treated group (G2). Therefore, it was confirmed that the hypoxic-non-treated INBS-ASC-Ther was effective in ameliorating the damaged liver (Table 1 and Figure 6).

**[Table 1]**

| Group | Collagen fiber occupied regions (%/mm²) | Degenerative hepatocyte numbers (cells/1000 cells) | Inflammatory cell numbers (cells/mm²) |
|---|---|---|---|
| G1 | 6.26+0.77 | 45.20+7.68 | 32.60+3.37 |
| G2 | 33.33±4.62⁺⁺ | 520.00±70.63⁺⁺ | 195.20±21.84⁺⁺ |
| G3 | 30.26±3.27 | 527.60±70.27 | 218.80±50.16 |
| G4 | 16.70±2.25* | 226.60±56.40* | 98.00±17.85** |

| | | | |
|---|---|---|---|
| (++: statistical difference between G1 and G2, P<0.01;*: statistically different from G2, P<0.05;**: statistically different from G2, P<0.01.) | | | |

From the above results, it was confirmed that there was no difference between the hypoxic-treated INBS-ASC-Ther group (G3) and the non-treated group (G2) in hematological and histopathological tests when INBS-ASC-Ther was subcutaneously injected once into SD rats with liver damage caused by DMN. In the hypoxic-non-treated INBS-ASC-Ther group (G4), a decrease of the liver damage indices was confirmed in the blood biochemical tests based on the day of autopsy, and a significant decrease of the liver damage indices was also confirmed in the histopathological tests. Therefore, improvement of liver dysfunction due to liver damage was confirmed in the hypoxic-non-treated INBS-ASC-Ther group (G4). The above results suggest that INBS-ASC-Ther can have an effect on improving liver function even if it is injected subcutaneously into the back, which is a site far away from the liver, a disease region.

### Example 3: Selection of micro RNA (miRNA) with antifibrotic effect

Through literature search using bioinformatics such as PubMed, miRNA base, and Cochrane, miRNAs reported to inhibit fibrosis were selected.

As a result, three miRNAs, miR-29 (SEQ. ID. NO: 1), miR-34 (SEQ. ID. NO: 2) and miR150 (SEQ. ID. NO: 3), were selected as micro RNAs with antifibrotic effect.

**[Table 2]**

| miRNA | Sequence (5'→3') | SEQ. ID. NO: |
|---|---|---|
| miR-29 (hsa-mir-20a) | AUGACUGAUUUCUUUUGGUGUUCAGAGUC AAUAUAAUUUUCUAGCACCAUCUGAAAUC GGUUAU | 1 |
| miR-34 (hsa-mir-34a) | GGCCAGCUGUGAGUGUUUCUUUGGCAGUG UCUUAGCUGGUUGUUGUGAGCAAUAGUAA GGAAGCAAUCAGCAAGUAUACUGCCCUAG AAGUGCUGCACGUUGUGGGGCCC | 2 |
| miR-150 (hsa-miR-150-5p) | UCUCCCAACCCUUGUACCAGUG | 3 |

### Example 4: Introduction of micro RNA into adipose-derived stem cells and concentration of medium

Adipose-derived stem cells (ASCs) were cultured in the same manner and conditions as described in Example 1. The cells were transfected with miR24, miR29, and miR150, respectively, and further cultured. Then, the micro RNA-introduced ASC culture medium was concentrated 25 times using an ultracentrifugal filter (Millipore Amicon).

### Example 5: Preparation of micro RNA-introduced ASCs contained in bioimplant

The micro RNA-introduced ASCs cultured in Example 4 were injected into TheraCyte™, a bioimplant.

Particularly, 1×10⁶ ASCs cultured for 24 hours in a hypoxia chamber were injected into TheraCyte (TheraCyte Inc., PD20.0, Ported TheraCyte® Device).

### Experimental Example 4: Confirmation of in vitro hepatocyte regeneration effect of adipose-derived stem cells introduced with micro RNA

### <4-1> Confirmation of hepatocyte regeneration effect in hepatocyte cell line (AML12)

The effect of regenerating hepatocytes was confirmed using the culture medium enriched with micro RNA-introduced ASCs prepared in Example 4.

Particularly, the ASC-enriched medium (NCM), the miR24-introduced ASC-enriched medium (MCM-24), the miR39-introduced ASC-enriched medium (MCM-39) and the miR150-introduced ASC-enriched medium (MCM-150) obtained in Example 4 were treated to the hepatocyte cell line (AML12, ATCC) treated with 50 mM thioacetamide or not treated, and the expression levels of the liver regeneration related factors PCNA, HGF and p-STAT3 were measured by Western blotting.

For Western blotting, AML12 cells were lysed using an EzRIPA lysis kit (Ato Corporation, Tokyo, Japan). Proteins were visualized with the primary antibody (1:1,000) at 4°C overnight, then with the HRP-conjugated secondary antibody (1:2,000) at 25°C for 1 hour. Along with the HRP-conjugated secondary anti-rabbit and HRP-conjugated anti-mouse IgGs (Cell Signaling, Beverly, Massachusetts), antibodies against hepatocyte growth factor (HGF) (Abcam, Cambridge, Massachusetts), proliferative cell nuclear antigen (PCNA), p-STAT3 and β-actin were used.

As a result, as shown in Figures 7a to 7d, it was confirmed that the expressions of p-STAT (Figure 7b), HGF (Figure 7c), and PCNA (Figure 7d), which are factors involved in liver regeneration, involved in cell proliferation were increased in the group treated with the micro RNA-introduced ASC-enriched culture medium compared to the control group treated with only TAA and the group treated with only ASCs.

### <4-2> Confirmation of liver fibrosis inhibitory effect in hepatic stellate cell line (LX2)

The effect of regenerating hepatocytes was confirmed using the culture medium enriched with micro RNA-introduced ASCs prepared in Example 4.

Particularly, the expression levels of α-SMA and TIMP were measured in the same manner and conditions as described in Experimental Example <4-1> except that 5 mM thioacetamide was treated, a hepatic stellate cell line (LX2, provided by Professor Jeong, Korea Advanced Institute of Science and Technology) was used, and the expression levels of α-SMA, a factor related to liver fibrosis, and TIMP, a fibrosis inhibitory factor, were measured.

As a result, as shown in Figures 8a to 8c, the expression of α-SMA, a factor related to liver fibrosis, was reduced (Figure 8b), and the expression of TIMP, a fibrosis inhibitory factor, was increased (Figure 8c) in the group treated with the micro RNA-introduced ASC-enriched culture medium compared to the control group treated with only TAA and the group treated with only ASCs.

### Experimental Example 5: Confirmation of liver fibrosis inhibitory effect in micro RNA treated group

Using the hepatic stellate cell line (LX2) of Experimental Example 4, the expressions of the factors related to liver fibrosis were measured in the group treated with miR24-introduced ASCs, the group treated with miR39-introduced ASCs, and the group treated with miR150-introduced ASCs, respectively.

### <5-1> Confirmation of liver fibrosis inhibitory effect in miR24-introduced ASC-treated group

The hepatic stellate cells (LX2) were treated or not treated with 5 mM thioacetamide, to which the miR24-introduced ASC-enriched medium prepared in Example 4 was treated. Then, the expression levels of MMP2, SMA, and TGF, which are factors related to liver fibrosis, were measured in the same manner and conditions as described in Experimental Example <4-1>.

As a result, as shown in Figures 9a to 9d, the expression levels of MMP2 (Figure 9b), α-SMA (Figure 9c) and TGF (Figure 9d), which are factors involved in liver fibrosis, were reduced in the miR24-introduced ASC-treated group compared to the control group treated with only TAA and the group treated with only ASCs. Therefore, it was confirmed that the miR24-introduced ASCs had an inhibitory effect on liver fibrosis.

### <5-2> Confirmation of liver fibrosis inhibitory effect in miR39-introduced ASC-treated group

The hepatic stellate cells (LX2) were treated or not treated with 5 mM thioacetamide, to which the miR39-introduced ASC-enriched medium prepared in Example 4 was treated. Then, the expression levels of MMP2 and α-SMA, which are factors related to liver fibrosis, were measured in the same manner and conditions as described in Experimental Example <4-1>.

As a result, as shown in Figures 10a to 10c, the expression levels of MMP2 (Figure 10b) and α-SMA (Figure 10c), which are factors involved in liver fibrosis, were reduced in the miR39-introduced ASC-treated group compared to the control group treated with only TAA and the group treated with only ASCs. Therefore, it was confirmed that the miR39-introduced ASCs had an inhibitory effect on liver fibrosis.

### <5-3> Confirmation of liver fibrosis inhibitory effect in miR150-introduced ASC-treated group

The hepatic stellate cells (LX2) were treated or not treated with 5 mM thioacetamide, to which the miR150-introduced ASC-enriched medium prepared in Example 4 was treated. Then, the expression levels of α-SMA and TIMP-1, which are factors related to liver fibrosis, were measured in the same manner and conditions as described in Experimental Example <4-1>.

As a result, as shown in Figures 11a to 11c, the expression of α-SMA, a factor related to liver fibrosis, was reduced (Figure 11b) and the expression of TIMP-1, a fibrosis inhibitory factor, was increased (Figure 11c) in the miR150-introduced ASC-treated group compared to the control group treated with only TAA and the group treated with only ASCs. Therefore, it was confirmed that the miR150-introduced ASCs had an inhibitory effect on liver fibrosis.

### Experimental Example 6: Measurement of secretion of VEGF, HGF and LDH of micro RNA-introduced ASCs contained in bioimplant

The liver regeneration effect was confirmed by measuring the secretion amounts of vascular epithelial growth factor (VEGF), hepatocyte growth factor (HGF) and lactate dehydrogenase (LDH) in the bioimplant comprising ASCs introduced with miR24, miR39 and miR150 prepared in Example 5.

Particularly, the secretion amounts of VEGF, HGF and LDH in the culture medium was measured by the method recommended by the manufacturer using an ELISA kit (Biolegend, San Diego, California).

As a result, as shown in Figure 12, it was confirmed that the secretion of VEGF, a growth factor, was increased in the bioimplant comprising miR24-introduced ASCs, the bioimplant comprising miR39-introduced ASCs and the bioimplant comprising miR150-introduced ASCs, compared to the bioimplant comprising ASCs. The effect of increasing the secretion amount of VEGF was the best in the bioimplant comprising miR150-introduced ASCs.

As shown in Figure 13, it was confirmed that the secretion of HGF, a growth factor, was increased in the bioimplant comprising miR24-introduced ASCs, the bioimplant comprising miR39-introduced ASCs and the bioimplant comprising miR150-introduced ASCs, compared to the bioimplant comprising ASCs. The effect of increasing the secretion amount of HGF was the best in the bioimplant comprising miR150-introduced ASCs.

As shown in Figure 14, it was confirmed that the secretion of LDH, a liver disease marker, was reduced in the bioimplant comprising miR24-introduced ASCs, the bioimplant comprising miR39-introduced ASCs and the bioimplant comprising miR150-introduced ASCs, compared to the bioimplant comprising ASCs. The effect of reducing the secretion amount of LDH was the best in the bioimplant comprising miR150-introduced ASCs.

### Experimental Example 7: Construction of liver fibrosis animal model

Liver fibrosis was induced by intraperitoneal administration of TAA (200 mg/kg) to 7-8 weeks old mice (BALB/c) three times a week for 5 weeks.

Six weeks after the induction of liver fibrosis, RT-PCR, Western blotting and immunostaining were performed with the mouse liver tissue to confirm the induction of liver fibrosis.

### Experimental Example 8: Confirmation of therapeutic effect of micro RNA-introduced ASCs contained in bioimplant in liver fibrosis animal model

The experimental groups were divided into ① the control group without any treatment (control), ② the group in which liver fibrosis was induced by treating thioacetamide (TAA) only (TAA only), ③ the group in which liver fibrosis was induced by treating TAA, and a bioimplant comprising ASCs was attached (TAA+ASC+Theracyte), ④ the group in which liver fibrosis was induced by treating TAA, and a bioimplant comprising miR24-introduced ASCs was attached (miR24-ASC+Theracyte+abdominal cavity), ⑤ the group in which liver fibrosis was induced by treating TAA, and a bioimplant comprising miR39-introduced ASCs was attached (miR39-ASC+Theracyte+abdominal cavity), ⑥ the group in which liver fibrosis was induced by treating TAA, and a bioimplant comprising miR150-introduced ASCs was attached (miR150-ASC+Theracyte+abdominal cavity), ⑦ the group in which liver fibrosis was induced by treating TAA, and a bioimplant comprising miR24-introduced ASCs was attached (miR24-ASC+Theracyte+subcutaneous), ⑧ the group in which liver fibrosis was induced by treating TAA, and a bioimplant comprising miR39-introduced ASCs was attached (miR39-ASC+Theracyte+subcutaneous), and ⑨ the group in which liver fibrosis was induced by treating TAA, and a bioimplant comprising miR150-introduced ASCs was attached (miR150-ASC+Theracyte+subcutaneous) .

Thereafter, the micro RNA-introduced ASCs contained in the bioimplant were engrafted on the epidermis of the liver fibrosis animal model, and the changes in liver enzyme levels, fibrosis indices, liver function regeneration and recovery factors were measured, and histopathological analysis was performed.

### <8-1> Measurement of changes in liver enzyme levels

In the experimental groups ① to ⑨, the levels of aspartate aminotransferase (AST) and alanine aminotransferase (ALT), which are indicators for determining liver disease as enzymes secreted by destruction of liver cells, were measured.

Particularly, serum was collected and the concentrations of the amino acid transferases aspartate aminotransferase (AST) and alanine aminotransferase (ALT), which are indicators of liver damage, were measured using an Idexx VetTest chemical analyzer.

As a result, the levels of AST and ALT were decreased in the groups in which the bioimplant comprising miR24, miR39 or miR150-introduced ASCs was administered intraperitoneally or subcutaneously, compared to the group in which liver fibrosis was induced by treating TAA alone. Therefore, it was confirmed that the reduction in liver function caused by TAA was recovered (Figures 15a to 15d).

### <8-2> Measurement of expressions of fibrosis indicators and recovery factors

The liver tissues were obtained from the experimental groups ① to ⑨, and the changes in the expression levels of MMP2, a fibrosis indicator, and TIMP-1, a liver fibrosis inhibitory factor, were confirmed by Western blotting.

Particularly, the liver tissues were lysed using an EzRIPA lysis kit (Ato Corporation, Tokyo, Japan), and after centrifugation at 12,000 rpm for 15 minutes, the supernatant was collected. The liver tissues were lysed with a lysis buffer (Roche Diagnostics Life Science, Indianapolis, Indiana, USA). The protein concentration in the lysate was measured using a Bradford reagent (Bio-Rad Laboratories, Hercules, CA, USA). The same amount of protein (30 *µ*g) per well was separated by SDS-PAGE, and electro-transferred to a nitrocellulose membrane blocked with 5% fat-free milk for 1 hour at room temperature. The plate was incubated overnight at 4°C with the primary antibodies (1:1,000) against TIMP2, procollagen, TGF-β1, HGF, PCNA, p-STAT3, Bcl-2 and β-actin. The plate was incubated for 1 hour at room temperature with the HRP-conjugated secondary anti-rabbit and anti-mouse IgGs (1:2,000) (Cell Signaling, Beverly, Massachusetts, USA). Certain immunocomplexes were detected using WesternBlotting Plus Chemiluminescence Reagent (Millipore).

As a result, the group in which a bioimplant comprising miR24, miR39 or miR150-introduced ASCs was administered showed an antifibrotic effect by adjusting the fibrosis factors by TAA by decreasing the expression of MMP2 and increasing the expression of TIMP1, compared to the group in which liver fibrosis was induced by treating TAA alone (Figure 16).

### <8-3> Histopathological tests

The liver was extracted from the animals of each experimental group and fixed in 10% neutral buffered formalin fixative. The fixed tissues were subjected to H&E staining, Mason's trichrome staining, and MMP2 immunochemical staining to perform morphological analysis.

Particularly, the extracted liver tissues were fixed with 10% formalin containing 0.1 M phosphate buffer (pH 7.2, Sigma), embedded in paraffin, and made into sections. The prepared paraffin sections (4 *µ*m thick) were de-waxed, hydrated, treated with 0.01% protease XXIV (Sigma) contained in phosphate-buffered saline at 37°C for 20 minutes, and stained with hematoxylin and eosin.

For Mason's trichrome staining, paraffin was removed from the paraffin-embedded tissue processed into 5 µm-thick sections, and the tissue was rehydrated in ethyl alcohol, washed with tap water, and re-fixed in Bouin solution at 56°C for 1 hour. After rinsing the tissue for 10 minutes in flowing tap water and staining for 10 minutes using Weigert' iron hematoxylin working solution, the slide was stained with Biebrich scarlet-acid fuchsin solution for 15 minutes and washed with tap water. The fragments were differentiated in a phosphomolybdic-phosphotungstic acid solution for 15 minutes, transferred to an aniline blue solution, and stained for 10 minutes. After brief rinsing in tap water, the slide was reacted with 1% acetic acid solution for 5 minutes. Semi-quantitative values for staining intensity were determined using digital image analysis (MetaMorph version 4.6r5, Universal Imaging Corp., Downingtown, PA, USA), testing at least 5 areas in each fragment under 400x magnification.

MMP2 was analyzed by immunohistochemical assay. For this, the formalin-fixed, paraffin-imbedded tissue sections were deparaffinized in xylene and rehydrated according to the grade of alcohol. The antigen was retrieved by standard procedure. The primary antibody according to the present invention is a PCNA antibody (1:300; Abcam). The tissue sections were counter-stained with hematoxylin. The number of MMP2 positive cells was determined by manually counting cells in 20 random fields of view under a laser-scanning microscope (Eclipse TE300, Nikon, Tokyo, Japan).

As a result of H&E and Mason's trichrome staining, as shown in Figure 17, the blue-stained area was reduced in the group in which a bioimplant comprising ASCs introduced with miR24, miR39 or miR150 was intraperitoneally administered, compared to the group in which liver fibrosis, the blue-stained area, was induced by treating TAA alone, indicating that the TAA-induced fibrin was reduced. As shown in Figure 18, the expression of MMP2 was decreased in the group in which a bioimplant comprising ASCs introduced with miR24, miR39 or miR150 was intraperitoneally administered, compared to the group in which the expression of MMP2, a liver fibrosis-inducing factor, was increased by treating TAA alone, indicating that the TAA-induced fibrin was reduced.

## Claims

1. A pharmaceutical composition for preventing or treating liver disease, comprising a bioimplant comprising mesenchymal stem cells.

2. The pharmaceutical composition for preventing or treating liver disease according to claim 1, wherein the mesenchymal stem cells are included in the bioimplant at the number of 1×10⁵ to 1×10⁹.

3. The pharmaceutical composition for preventing or treating liver disease according to claim 1, wherein the liver disease is any one or more selected from the group consisting of hepatitis, liver fibrosis and cirrhosis.

4. The pharmaceutical composition for preventing or treating liver disease according to claim 1, wherein the mesenchymal stem cells are the mesenchymal stem cells into which miRNA (micro RNA) has been introduced.

5. The pharmaceutical composition for preventing or treating liver disease according to claim 4, wherein the miRNA is miR24, miR39 or miR150.

6. The pharmaceutical composition for preventing or treating liver disease according to claim 4, wherein the composition increases the expression of PCNA, HGF, p-STAT or TIMP.

7. The pharmaceutical composition for preventing or treating liver disease according to claim 4, wherein the composition reduces the expression of α-SMA, MMP or TGF.

8. The pharmaceutical composition for preventing or treating liver disease according to claim 4, wherein the composition increases the secretion of vascular endothelial growth factor (VEGF) or hepatocyte growth factor (HGF).

9. The pharmaceutical composition for preventing or treating liver disease according to claim 4, wherein the composition reduces the secretion of lactate dehydrogenase (LDH).

10. The pharmaceutical composition for preventing or treating liver disease according to claim 1 or claim 4, wherein the composition reduces the levels of AST (aspartate aminotransferase) and ALT (alanine aminotransferase).

11. The pharmaceutical composition for preventing or treating liver disease according to claim 1, wherein the composition reduces the ratio of an area occupied by collagen fibers in liver parenchyma.

12. The pharmaceutical composition for preventing or treating liver disease according to claim 1, wherein the composition reduces the number of degenerate hepatic cells.

13. The pharmaceutical composition for preventing or treating liver disease according to claim 1, wherein the composition reduces the number of infiltrated inflammatory cells.

14. The pharmaceutical composition for preventing or treating liver disease according to any one of claims 1 to 9 and 11 to 13, wherein the composition is injected subcutaneously or intraperitoneally.

15. The pharmaceutical composition for preventing or treating liver disease according to claim 14, wherein the composition is injected subcutaneously in the back.

16. A method of preventing, ameliorating or treating liver disease, comprising a step of administering a bioimplant comprising mesenchymal stem cells to a subject.

17. A use of a bioimplant comprising mesenchymal stem cells for the manufacture of a medicament for the prevention, amelioration or treatment of liver disease.
